# EUROPEAN PATENT SPECIFICATION

(11) **EP 3 089 733 B1**
(45) Date of publication and mention of the grant of the patent: **11.07.2018**
(21) Application number: 14876936.7
(22) Date of filing: 01.12.2014
(51) Int. Cl.: A61K 8/365, A61Q 15/00, A61K 8/06

(54) **ANTIPERSPIRANT COMPOSITION WITH A CITRIC ACID STAIN REDUCING AGENT**
SCHWEISSHEMMENDE ZUSAMMENSETZUNG MIT EINEM ZITRONENSÄUREMITTEL ZUR VERRINGERUNG VON FLECKEN
COMPOSITION ANTI-TRANSPIRATION COMPRENANT UN AGENT RÉDUCTEUR DE TACHES À L'ACIDE CITRIQUE

(30) Priority: 30.12.2013 US 201314143034
(43) Date of publication of application: 09.11.2016
(73) Proprietor: Henkel IP & Holding GmbH, 40589 Düsseldorf (DE)
(72) Inventor: GE, Haiyan, Valencia, California 91381 (US); TSOTSOS, Amy, Scottsdale, Arizona 85253 (US); BANOWSKI, Bernhard, 40597 Duesseldorf (DE); LESTER, Aleidatje M., Phoenix, Arizona 85086 (US)
(74) Representative: Henkel IP Department
(86) International application number: PCT/US2014/067861
(87) International publication number: WO 2015/102785

(56) References cited:
- WO-A1-2011/077144
- WO-A1-2013/116217
- DE-A1-102010 034 389
- US-A1- 2011 038 822
- US-A1- 2012 304 397
- US-A1- 2013 160 788
- US-A1- 2013 164 236
- US-A1- 2013 164 352

## Description

### CROSS-REFERENCE TO RELATED APPLICATIONS

The present application claims priority to U. S. Utility Application 14/143,034 filed December 30, 2013 and entitled "ANTIPERSPIRANT COMPOSITION WITH A CITRIC ACID STAIN REDUCING AGENT", which is incorporated herein.

### FIELD OF THE INVENTION

The present invention generally relates to a composition to reduce precipitate formation on fabric, and more particularly relates to an antiperspirant composition with a citric acid stain reducing agent.

### BACKGROUND OF THE INVENTION

Antiperspirants are personal care products that are used to prevent or reduce perspiration and odors resulting from perspiration, particularly perspiration from a person's underarm. Such antiperspirants may interact with sweat, sebum soils, and detergent ingredients to form precipitates on fabric. The precipitates may turn yellow with time leaving stains on fabric such as clothing. Such staining may be unsightly and embarrassing.

Accordingly, it is desirable to have an antiperspirant composition that results in less staining of a fabric surface. Additionally, it is desirable to have an antiperspirant composition that has improved application aesthetics and increased antiperspirant efficacy. Furthermore, other desirable features and characteristics of the present invention will become apparent from the subsequent detailed description of the invention and the appended claims, taken in conjunction with the accompanying drawings and this background of the invention.
WO2013/116217 A1 discloses an antiperspirant water-in-oil emulsion composition comprising water phase, oil phase and an active antiperspirant.
Antiperspirant water-in-oil emulsion compositions are also disclosed in DE 10 2010 034389 A1. These may comprise citric acid (paragraph [0166] and an active antiperspirant agent.

### BRIEF SUMMARY OF THE INVENTION

An antiperspirant composition with citric acid as a stain reducing agent includes a water-in-oil emulsion. The water-in-oil emulsion includes an oil-phase component disposed within the water-in-oil emulsion and a water-phase component disposed within the water-in-oil emulsion. The water-phase component includes an active antiperspirant ingredient. The water-phase component also includes citric acid stain as a reducing agent forming between 0.9 to 4.0 weight percent of the water-in-oil emulsion in which the water-in-oil emulsion has a pH of between 2.0 and 3.0.

An antiperspirant product with citric acid as a stain reducing agent includes a container and a water-in-oil antiperspirant emulsion housed within the container. The water-in-oil antiperspirant emulsion includes an oil-phase component disposed within the water-in-oil emulsion and a water-phase component disposed within the water-in-oil emulsion. The water-phase component includes an active antiperspirant ingredient. The water-phase component also includes a citric acid stain reducing agent forming between 0.9 to 4.0 weight percent of the water-in-oil emulsion.

An antiperspirant composition with citric acid as a stain reducing agent includes a water-in-oil emulsion. The water-in-oil emulsion includes between 25.0 to 35.0 weight percent of an oil-phase component disposed within the water-in-oil emulsion. The water-in-oil emulsion also includes between 65.0 to 75.0 weight percent of a water-phase component disposed within the water-in-oil emulsion. The water-phase component includes an active antiperspirant ingredient. The water-phase component also includes citric acid as a stain reducing agent to protect the active antiperspirant ingredient an interaction with a detergent, forming between 0.9 to 4.0 weight percent of the water-in-oil emulsion.

### BRIEF DESCRIPTION OF THE DRAWINGS

The present invention will hereinafter be described in conjunction with the following drawing figures, wherein like numerals denote like elements, and

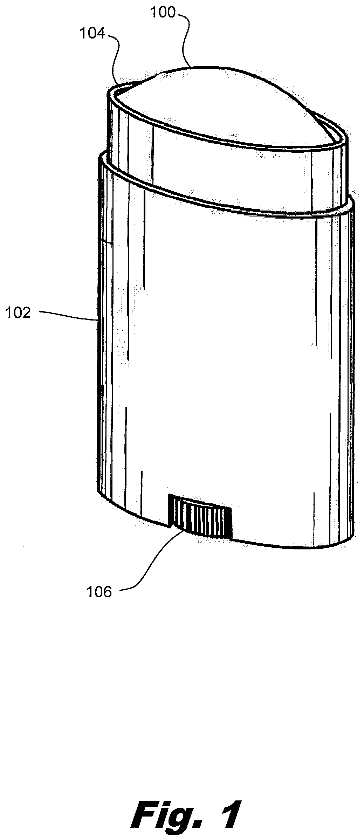

FIG. 1 is a diagram of an example of container for applying an antiperspirant composition with a citric acid stain reducing agent according to the principles described herein.

### DETAILED DESCRIPTION OF THE INVENTION

The following detailed description of the invention is merely exemplary in nature and is not intended to limit the invention or the application and uses of the invention. Furthermore, there is no intention to be bound by any theory presented in the preceding background of the invention or the following detailed description of the invention.

As described above, perspiration is a common occurrence in most individual's lives. Perspiration itself and odors emanating from the perspiration can be uncomfortable, unsightly, and embarrassing. To combat perspiration, an individual may use an antiperspirant substance to reduce the perspiration itself and odors emanating from the perspiration. Such antiperspirant substances include an active antiperspirant ingredient. The active antiperspirant ingredient may reduce perspiration, perspiration odor, perspiration wetness, or combinations thereof. However, in some examples, the active antiperspirant ingredient may interact with detergent used to launder clothing. The interaction may result in precipitates that form on the clothing fabric. Such precipitates may also form due to an interaction between the active antiperspirant ingredient and other ingredients in the antiperspirant substance, sweat and sebum soils. Such precipitates may turn yellow in time, leaving a stain on the fabric. For example, antiperspirant compositions applied to an underarm may interact with laundry detergent to form yellow stains on the armpit region of a shirt. These yellow stains may be both unsightly and embarrassing for an individual. The stains may also reduce the lifecycle of an article of clothing.

Antiperspirants may come in various forms. For example, some antiperspirants are invisible solid stick antiperspirants, clear gel antiperspirants, and roll-on antiperspirants. Some of these antiperspirant types may exhibit application aesthetics that lead to a less than satisfactory user experience. For example, some antiperspirant substances may feel "sticky" and may be difficult to apply. Moreover, various types of antiperspirant may leave residues on the skin surface during use.

Accordingly, the principles described herein provide a composition for reducing precipitate formation on fabric, which may result in reduced yellow staining of the fabric. For example, an antiperspirant composition as will be described below may include citric acid as a stain reducing agent in a water-in-oil emulsion antiperspirant composition. In some examples, the citric acid may protect an active antiperspirant ingredient. Such protection may reduce the ability of the active antiperspirant ingredient to polymerize and may reduce the ability of the active antiperspirant ingredient to interact with laundry detergent, sweat, sebum soils, or other compounds. The reduced interaction may reduce the propensity of particulate formation, thus reducing yellow staining. The quantity of citric acid present in the antiperspirant composition described herein may be a unique aspect of the invention. Additionally, the use of the citric acid as an protectant of the active antiperspirant ingredient may also be a unique aspect of the present invention.

Additionally, the principles described herein provide an antiperspirant composition that has improved aesthetics such as a less sticky feel and has an improved gliding motion during application. For example, the antiperspirant composition as described below may be a water-in-oil emulsion that may leave less white residue and may provide pleasing application aesthetics such as a "gliding" motion.

Still further, an antiperspirant composition with citric acid may improve antiperspirant efficacy. For example, as described above, an antiperspirant composition with citric acid may reduce the active antiperspirant ingredient molecular weight. Active antiperspirant ingredients with a lower molecular weight, or low polymer size may provide more intense sweat reduction.

Accordingly, the antiperspirant composition disclosed herein may prevent precipitate formation on fabric, provide improved aesthetics, and exhibit enhanced antiperspirant efficacy to leave the consumer with an overall satisfactory experience.

Turning now to the figures, FIG. 1 is a diagram of a container (102) for applying an antiperspirant composition (100) with a citric acid stain reducing agent according to the principles described herein. In this example, the antiperspirant composition (100) may be held within a container (102) that has an opening (104) that allows the antiperspirant composition (100) to be pushed out of the container (102) and onto a surface. For example, the antiperspirant composition (100) may extend beyond the opening (104) of the container (102) and be applied to skin. As indicated in FIG. 1, the antiperspirant composition (100) may be approximately dome-shaped and may be applied to skin. For example, the antiperspirant composition (100) may be applied to an underarm. The container (102) may also include a dispenser to dispense the antiperspirant composition (100). For example, the antiperspirant composition (100) can be pushed out of the opening (104) by rotating a dial (106), or a knob, positioned at the bottom of the container (102). As the dial (106) is rotated, a platform internal to the container (102) moves up and pushes the solid antiperspirant composition (100) with it.

Any appropriate type of container (102) may be used to hold and apply the antiperspirant composition (100) to skin. For example, a container (102) with an oval cross section may be used to hold the antiperspirant composition (100). Also, a container (102) with a more cylindrical cross section may be used in some examples. The container (102) may have any appropriate shape in accordance with the principles described herein. In some examples, the container (102) may include a protective cover.

During use, a top of the container (102) may be removed and the antiperspirant composition (100) exposed. Via the container (102), a user may apply the antiperspirant composition (100) to skin by rubbing the antiperspirant composition (100) across the skin.

The antiperspirant composition (100) may be a water-in-oil emulsion. In other words, the antiperspirant composition (100) may include a number of phase components that are otherwise immiscible. A water-in-oil emulsion antiperspirant composition (100) may be beneficial in that it may leave less white residue on a skin surface and may improve application aesthetics. In other words, the water-in-oil antiperspirant composition (100) may feel smoother during application.

The water-in-oil emulsion antiperspirant composition (100) may include a water-phase component and an oil-phase component. The oil-phase component may include a number of ingredients to improve the application aesthetics of the antiperspirant composition (100) or to improve other characteristics of the antiperspirant composition. For example, the oil-phase component may include an emollient. An emollient may refer to any mixture or chemical compound that is configured to make layers of the skin softer and more pliable. Accordingly, the oil-phase component may include any compound or mixture that is configured to make layers of skin softer and more pliable.

The oil-phase component may also include a number of carriers. A carrier may be any compound or mixture configured to deliver other ingredients. Accordingly, the oil-phase component may include any compound or mixture that is configured to deliver other ingredients to the skin. The oil-phase component may also include a number of structurants. A structurant may be any compound or mixture configured to give shape, or structure to the antiperspirant composition (100). Accordingly, the oil-phase component may include any compound or mixture that is configured to give shape, or structure to the antiperspirant composition. The oil-phase component may include a number of emulsifiers. An emulsifier may be any compound or mixture that is configured to provide stability to a water-in-oil emulsion. Accordingly, the oil-phase component may include any compound or mixture that is configured to stabilize the water-in-oil emulsion. Non-limiting, specific examples of emollients, carriers, structurants, and emulsifiers are given below in Table (3). In some examples, the oil-phase component may form between 25.0 and 35.0 weight percent of the antiperspirant composition (100), such as 29.5 weight percent.

The water-in-oil emulsion antiperspirant composition (100) may also include a water-phase component. The water-phase component may include an active antiperspirant ingredient. The active antiperspirant ingredient may be any active ingredient that may reduce perspiration, perspiration odors, wetness resulting from perspiration, or combinations thereof. In some examples, the active antiperspirant ingredient may be a salt that reduces perspiration by diffusing through the sweat ducts of sweat glands and may plug the sweat glands by precipitation.

Examples of active antiperspirant ingredients include astringent water-soluble inorganic and organic salts of aluminum, zirconium and zinc or any mixtures of these salts. Examples of active antiperspirant ingredients include, aluminum zirconium techlorohydrex, aluminum halides, aluminum chlorohydrates, aluminum hydroxyhalides, zirconyl oxyhalides, zirconyl hydroxyhalides, aluminum dichlorohydrate, aluminum-zirconium octachlorohydrate, aluminum sesquichlorohydrate, aluminum chlorohydrex propylene glycol complex, aluminum dichlorohydrex propylene glycol complex, aluminum sesquichlorohydrex propylene glycol complex, aluminum chlorohydrex polyethylene glycol complex, aluminum dichlorohydrex polyethylene glycol complex, aluminum sesquichlorohydrex polyethylene glycol complex, aluminum-zirconium trichlorohydrate, aluminum zirconium tetrachlorohydrate, aluminum zirconium pentachlorohydrate, aluminum zirconium octachlorohydrate, aluminum zirconium trichlorohydrex glycine complex, aluminum zirconium tetrachlorohydrex glycine complex, aluminum zirconium pentachlorohydrex glycine complex, aluminum zirconium octachlorohydrex glycine complex, zirconium chlorohydrate, aluminum chloride, and combinations thereof.

While the above examples have been described with reference to specific types of active antiperspirant ingredients, any appropriate antiperspirant ingredient may be used in accordance with the principles described herein. For example, the active antiperspirant ingredient may be used to prevent perspiration and perspiration odors, inhibit the formation of perspiration and perspiration odors, or otherwise contribute to reducing perspiration. Further, the active antiperspirant ingredients may include multiple types of active antiperspirant ingredients that reduce perspiration and perspiration odors. In such examples, each of the active antiperspirant ingredients may perform different functions, perform overlapping functions, perform the same functions, or combinations thereof.

The antiperspirant composition (100) may include the active antiperspirant ingredient in an amount that provides an antiperspirant effect. For example, the active antiperspirant ingredient may form between 12.0 and 20.0 weight percent of the antiperspirant composition (100), such as 19.0 weight percent. In some examples, the active antiperspirant ingredient may have a pH of between 3.0 and 4.5.

The water-phase component of the antiperspirant composition (100) may also include an active carrier ingredient to distribute the active antiperspirant ingredient throughout the water-phase component. The active carrier ingredient may include any ingredient that distributes the active antiperspirant ingredient throughout the water-phase component. For example, the active carrier ingredient may be propylene glycol that distributes the active antiperspirant salt throughout the water-phase component. The carrier ingredient may form between 2.0 and 20.0 weight percent of the antiperspirant composition (100), such as 5.0 weight percent.

In some examples, the water-phase component may include a citric acid stain reducing agent. The citric acid stain reducing agent may aid in prevention of precipitate formation on fabric. More specifically, the citric acid stain reducing agent may protect an active antiperspirant ingredient. For example, citric acid particles may protect an active antiperspirant polymer, and prevent the active antiperspirant polymer from reacting with a detergent, sweat, sebum soils, or other ingredients in the antiperspirant composition (100). Accordingly, the protectant citric acid may prevent the formation of precipitates on fabric by reducing the probability of an interaction of the active antiperspirant ingredient with detergent, sweat, sebum soils, other ingredients within the antiperspirant composition (100) as the active antiperspirant ingredient is not in contact with these compounds.

The use of the citric acid as a protectant for the active antiperspirant ingredient and other ingredients, detergent, sweat, sebum soils or other compounds may be a unique aspect of the antiperspirant composition (100) as general practice in the industry may not rely on citric acid in this fashion. An antiperspirant composition (100) with citric acid may be beneficial in that the citric acid protectant may prevent the active antiperspirant ingredient from contacting other compounds that may lead to the formation of precipitates, and corresponding yellow stains, on fabric. Additionally, the citric acid may modify the polymer distribution of Aluminum salt polymers in an aqueous solution as indicated in Table (1).

Table (1) indicates a shift in the polymer distribution. More specifically, Table (1) indicates a shift to more short chain polymers which may be more active. A chromatogram show peaks for more long chain polymers (peak 1-3), peaks for mid chain polymers (peak 6-8) and short chain polymers (peak 9-12). Table (1) indicates the peak distribution based on the molecular weight of the polymers of an active antiperspirant ingredient in a number of antiperspirant compositions. More particularly, Table (1) represents information of data gathered from a chromatogram that indicates the polymer length of an active antiperspirant ingredient as used in a typical emulsion antiperspirant composition and the molecular weight of an active antiperspirant ingredient as used in a water-in-oil emulsion antiperspirant composition (100) with a citric acid stain reducing agent as disclosed herein. The data indicated in Table (1) was gathered using high-performance liquid chromatography techniques.

**Table (1)**

| Peak | 1 | 2&3 | 4&5 | 6&7 | 8 | 9 | 10&11 | 12 | >12 |
|---|---|---|---|---|---|---|---|---|---|
| Active Antiperspirant Ingredient | 1 | 1 | | 45 | 5 | | 36 | 2 | 10 |
| Antiperspirant Composition without Citric Acid | | 1 | | 45 | 4 | | 36 | | 14 |
| Antiperspirant Composition with 1.5% Citric Acid | 2 | 4 | 6 | 30 | 4 | | 43 | | 11 |

In Table (1), the antiperspirant composition (100) with 1.5% citric acid may be an example of the antiperspirant composition described herein, which may have a pH of between 2.0 and 3.0. The "peaks" as indicated in the first row of Table (1) may refer to a polymer chain length as detected during high-performance liquid chromatography for an antiperspirant active ingredient. For example, columns "1" and "2&3" may indicate long-chain polymers. By comparison column "6&7" and "8" may indicate mid-chain polymers, and columns "9," "10&11," "12," and ">12" may indicate short-chain polymers. In Table (1), the numbers in rows 2-4 (i.e., the rows for an active antiperspirant ingredient, an antiperspirant composition without citric acid, and an antiperspirant composition with 1.5% citric acid), may indicate a percentage of polymers within the composition that correspond to that particular polymer chain length. The number may indicate an area under the curve within that peak range. For example, approximately 43% of the area under the curve for an antiperspirant composition (100) with 1.5% citric acid is found under the peaks numbered 10 and 11. Stated another way, approximately 43% of the polymers in an antiperspirant composition with 1.5% citric acid are short-range polymers corresponding to the peak numbers 10 and 11.

In general, antiperspirant active ingredients with less efficacy contain mid-chain polymers and long-chain polymers in a higher percentage and more efficient antiperspirant active ingredients contain more mid-chain polymers and short-chain polymers. Also, in general, long-chain polymers are generally more stable than mid-chain polymers and short-chain polymers. Accordingly, an antiperspirant composition with citric acid may stabilize the polymers in a solution.

As indicated by Table (1), the antiperspirant composition (100) as disclosed herein, and containing 1.5% citric acid may have more short-chain active antiperspirant ingredient polymers than the antiperspirant composition without citric acid. In other words, the antiperspirant composition (100) as disclosed herein, with 1.5% citric acid may include an active antiperspirant ingredient with a lower molecular weight. This is indicated by the greater number in column "10&11." As such, the distribution of polymers is stabilized by shifting to more short-chain polymers with the addition of the citric acid. In other words, the citric acid may stabilize the polymer distribution of the active antiperspirant ingredient. Such stabilization may be an enhancement of the mid and short chain polymer distribution.

Similarly, as the antiperspirant composition (100) with citric acid has shorter chain polymer active antiperspirant ingredients, the antiperspirant composition (100) with citric acid may increase the antiperspirant efficacy. Accordingly, an antiperspirant composition (100) with a citric acid stain reducing agent may be beneficial by reducing active antiperspirant polymer growth which may create a stronger sweat plug.

The citric acid stain reducing agent may form between 0.9 to 4.0 weight percent of the water-in-oil-emulsion, such as 1.5 weight percent. Including citric acid in an antiperspirant composition (100) within this weight percent range may be a unique aspect of the antiperspirant composition (100) as general practice within the field does not implement citric acid concentrations in this amount. In some examples, the water-phase component may form between 65.0 and 75.0 weight percent of the antiperspirant composition (100), such as 69.0 weight percent.

The antiperspirant composition (100) may include other ingredients such as anti-bacterial additives, dyes, antioxidants, and moisturizers among other additive to achieve a desired purpose or function. Specifically, the antiperspirant composition (100) may include fragrances to provide the antiperspirant composition (100) with a pleasant smell. In some examples, the fragrance may form between 0.05 and 5.0 weight percent of the antiperspirant composition (100), such as 1.5 weight percent.

The antiperspirant composition (100) may include a deodorant to reduce odors emanating from perspiration. Examples of deodorant additives include any agent to neutralize, suppress, or mask perspiration odor. The antiperspirant composition (100) may also include antibacterials and enzyme-inhibiting deodorant active.

In addition to the aforementioned components, further additives may be included in the antiperspirant composition for various purposes including additives that cause the antiperspirant composition to exhibit long-lasting fragrance, odor protection, bacteria control, and/or another desired purpose and/or function. Specific examples of additional such additives include, but are not limited to, fragrances, including encapsulated fragrances; skin conditioners; dyes; pigments; preservatives; antioxidants; moisturizers; and the like.

Tables (2)-(4) present an example of an antiperspirant composition (100) as described herein. More specifically, the antiperspirant composition (100) may include a water-phase component that includes the following ingredients in the corresponding weight percentages as indicated in Table (2).

**Table (2)**

| Ingredient | Weight Percent (%) |
|---|---|
| Antiperspirant Active Ingredient | 62.5 |
| Propylene Glycol | 5.0 |
| Citric Acid | 0.9-4.0 |

As used in Table (2), propylene glycol may be the carrier of the active antiperspirant ingredient. As indicated in Table (2), the water-phase component may also include citric acid. The antiperspirant composition (100) may include an oil-phase component that includes the following ingredients in the corresponding weight percentages as indicated in Table (3).

**Table (3)**

| Ingredient | Weight Percent (%) |
|---|---|
| C12-15 Alkyl Benzoate | 8.035 |
| 2 centistokes (cst) Dimethicone | 6.432 |
| 5 centistokes (cst) Dimethicone | 1.5680 |
| Polyethylene component A | 10.942 |
| Polyethylene component B | 1.223 |
| Cetyl PEG/PPG-10/1 Dimethicone | 0.998 |
| Bis-PEG/PPG-14/14 Dimethicone | .202 |
| Synthetic Wax | 0.1 |

As used in Table (3), C12-15 alkyl benzoate may be an emollient, dimethicone at 2 centistokes and dimethicone at 5 centistokes may be emollients, polyethylene component A and polyethylene component B may be structurants, cetyl PEG/PPG-10/1 dimethicone and bis-PEG/PPG-14/14 dimethicone may be emulsifiers and synthetic wax may be another structurant. While Tables (2) and (3) make particular reference is made to specific compounds, other compounds that provide similar functionality as described herein may be implemented.

Table (4) indicates an overall antiperspirant composition (100) including the water-phase component as indicated in Table (2) and the oil-phase component as indicated in Table (3).

As indicated in Table (4), the antiperspirant composition (100) may include a water-phase component as indicated in Table (2), an oil-phase component as indicated in Table (3) and a fragrance in the corresponding weight percentages as indicated in Table (4).

**Table (4)**

| Ingredient | Weight Percent (%) |
|---|---|
| Water-phase Component | 69.0 |
| Oil-phase Component | 29.5 |
| Fragrance | 1.5 |

The antiperspirant composition (100) may be formed by combining the ingredients that make up the water-phase and combining the ingredients that make-up the oil-phase. The different phases may then be combined and mixed. Other ingredients such as fragrance may be added and the mixture homogenized.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention, it being understood that various changes may be made in the function and arrangement of elements described in an exemplary embodiment without departing from the scope of the invention as set forth in the appended claims and their legal equivalents.

## Claims

1. An antiperspirant composition with citric acid as a stain reducing agent, comprising:
a water-in-oil emulsion comprising:
an oil-phase component disposed within the water-in-oil emulsion; and
a water-phase component disposed within the water-in-oil emulsion, the water-phase component comprising:
an active antiperspirant ingredient; and
citric acid as a stain reducing agent forming between 0.9 to 4.0
weight percent of the water-in-oil emulsion,
in which the water-in-oil emulsion has a pH of between 2.0 and 3.0.

2. The antiperspirant composition of claim 1, in which the citric acid stain reducing agent protects the active antiperspirant ingredient.

3. The antiperspirant composition of claim 1, in which the citric acid stain reducing agent forms 1.5 weight percent of the water-in-oil emulsion.

4. The antiperspirant composition of claim 1, in which the citric acid stain reducing agent prevents polymerization of the active antiperspirant ingredient.

5. The antiperspirant composition of claim 1, in which the citric acid stain reducing agent stabilizes polymer distribution of the active antiperspirant ingredient, enhances the polymer distribution of the active antiperspirant ingredient, or combinations thereof.

6. The antiperspirant composition of claim 1, in which the oil-phase component comprises an emollient, a number of carriers, a number of structurants, a number of emulsifiers, or combinations thereof.

7. The antiperspirant composition of claim 1, in which the active antiperspirant ingredient is an aluminum salt with a pH of between 3.0 and 4.5.

8. An antiperspirant product with citric acid as a stain reducing agent comprising:
a container; and
a water-in-oil antiperspirant emulsion housed within the container, the water-in-oil antiperspirant emulsion comprising:
an oil-phase component disposed within the water-in-oil antiperspirant emulsion; and
a water-phase component disposed within the water-in-oil antiperspirant emulsion, the water-phase component comprising:
an active antiperspirant ingredient; and
citric acid as a stain reducing agent forming between 0.9 to 4.0 weight percent of the water-in-oil emulsion, in which the water-in-oil emulsion has a pH of between 2.0 and 3.0.

9. The antiperspirant product of claim 8, in which the citric acid stain reducing agent protects the active antiperspirant ingredient.

10. The antiperspirant product of claim 8, wherein the container comprises a dispenser to push the solid antiperspirant composition out of the container.

11. The antiperspirant product of claim 8, in which the citric acid stain reducing agent is configured to reduce the formation of precipitate on fabric.

12. The antiperspirant product of claim 8, further comprising a dye, fragrance, or combinations thereof.

13. The antiperspirant product of claim 8, in which the citric acid stain reducing agent forms 1.5 weight percent of the water-in-oil emulsion.

14. An antiperspirant composition of claim 1 with citric acid as a stain reducing agent, comprising:
a water-in-oil emulsion comprising:
between 25.0 to 35.0 weight percent of an oil-phase component disposed within the water-in-oil emulsion; and
between 65.0 to 75.0 weight percent of a water-phase component disposed within the water-in-oil emulsion, the water-phase component comprising:
an active antiperspirant ingredient; and
citric acid as a stain reducing agent to protect the active antiperspirant ingredient forming between 0.9 to 4.0 weight percent of the water-in-oil emulsion,
in which the water-in-oil emulsion has a pH of between 2.0 and 3.0.

15. The antiperspirant composition of claim 17, in which the citric acid reducing agent is configured to prevent an interaction between the active antiperspirant ingredient and a fabric detergent.

16. The antiperspirant composition of claim 17, in which the oil-phase component comprises c12-15 alkyl benzoate, dimethicone, polyethylene, cetyl PEG/PPG-10/dimethicone, bis-PEG/PPG-14/14 dimethicone/dimethicone, a polymer or combinations thereof.

## Patentansprüche

1. Antitranspirationszusammensetzung mit Citronensäure als Fleckenreduktionsmittel, umfassend:
eine Wasser-in-ÖI-Emulsion, umfassend:
einen in der Wasser-in-ÖI-Emulsion angeordneten Ölphasenbestandteil und
einen in der Wasser-in-ÖI-Emulsion angeordneten Wasserphasenbestandteil, wobei der Wasserphasenbestandteil Folgendes umfasst:
einen Antitranspirationswirkstoff; und
Citronensäure als Fleckenreduktionsmittel, das zwischen 0,9 und 4,0 Gewichtsprozent der Wasser-in-ÖI-Emulsion bildet,
wobei die Wasser-in-ÖI-Emulsion einen pH-Wert zwischen 2,0 und 3,0 aufweist.

2. Antitranspirationszusammensetzung nach Anspruch 1, wobei das Citronensäurefleckenreduktionsmittel den Antitranspirationswirkstoff schützt.

3. Antitranspirationszusammensetzung nach Anspruch 1, wobei das Citronensäurefleckenreduktionsmittel 1,5 Gewichtsprozent der Wasser-in-ÖI-Emulsion bildet.

4. Antitranspirationszusammensetzung nach Anspruch 1, wobei das Citronensäurefleckenreduktionsmittel eine Polymerisation des Antitranspirationswirkstoffs verhindert.

5. Antitranspirationszusammensetzung nach Anspruch 1, wobei das Citronensäurefleckenreduktionsmittel die Polymerverteilung des Antitranspirationswirkstoffs stabilisiert, die Polymerverteilung des Antitranspirationswirkstoffs verbessert oder Kombinationen davon.

6. Antitranspirationszusammensetzung nach Anspruch 1, wobei der Ölphasenbestandteil einen Weichmacher, eine Vielzahl von Trägern, eine Vielzahl von Strukturbildnern, eine Vielzahl von Emulgiermitteln oder Kombinationen davon umfasst.

7. Antitranspirationszusammensetzung nach Anspruch 1, wobei es sich bei dem Antitranspirationswirkstoff um ein Aluminiumsalz mit einem pH-Wert zwischen 3,0 und 4,5 handelt.

8. Antitranspirationsprodukt mit Citronensäure als Fleckenreduktionsmittel, umfassend:
Behälter; und
eine in dem Behälter untergebrachte Wasser-in-ÖI-Antitranspirationsemulsion, wobei die Wasser-in-ÖI-Antitranspirationsemulsion Folgendes umfasst:
einen in der Wasser-in-ÖI-Emulsion angeordneten Ölphasenbestandteil und
einen in der Wasser-in-ÖI-Emulsion angeordneten Wasserphasenbestandteil, wobei der Wasserphasenbestandteil Folgendes umfasst:
einen Antitranspirationswirkstoff; und
Citronensäure als Fleckenreduktionsmittel, das zwischen 0,9 und 4,0 Gewichtsprozent der Wasser-in-ÖI-Emulsion bildet, wobei die Wasser-in-ÖI-Emulsion einen pH-Wert zwischen 2,0 und 3,0 aufweist.

9. Antitranspirationsprodukt nach Anspruch 8, wobei das Citronensäurefleckenreduktionsmittel den Antitranspirationswirkstoff schützt.

10. Antitranspirationsprodukt nach Anspruch 8, wobei der Behälter einen Spender umfasst, um die feste Antitranspirationszusammensetzung aus dem Behälter zu drücken.

11. Antitranspirationsprodukt nach Anspruch 8, wobei das Citronensäurefleckenreduktionsmittel dazu ausgelegt ist, die Bildung von Niederschlag auf Stoff zu verhindern.

12. Antitranspirationsprodukt nach Anspruch 8, ferner umfassend eine Farbe, einen Duft oder Kombinationen davon.

13. Antitranspirationsprodukt nach Anspruch 8, wobei das Citronensäurefleckenreduktionsmittel 1,5 Gewichtsprozent der Wasser-in-ÖI-Emulsion bildet.

14. Antitranspirationszusammensetzung nach Anspruch 1 mit Citronensäure als Fleckenreduktionsmittel, umfassend:
eine Wasser-in-ÖI-Emulsion, umfassend:
zwischen 25,0 und 35,0 Gewichtsprozent eines in der Wasser-in-ÖI-Emulsion angeordneten Ölphasenbestandteils und
zwischen 65,0 und 75,0 Gewichtsprozent eines in der Wasser-in-ÖI-Emulsion angeordneten Wasserphasenbestandteils, wobei der Wasserphasenbestandteil Folgendes umfasst:
einen Antitranspirationswirkstoff; und
Citronensäure als Fleckenreduktionsmittel, um den Antitranspirationswirkstoff zu schützen, der zwischen 0,9 und 4,0 Gewichtsprozent der Wasser-in-ÖI-Emulsion bildet, wobei die Wasser-in-Öl-Emulsion einen pH-Wert zwischen 2,0 und 3,0 aufweist.

15. Antitranspirationszusammensetzung nach Anspruch 17, wobei das Citronensäurereduktionsmittel dazu ausgelegt ist, eine Interaktion zwischen dem Antitranspirationswirkstoff und einem Waschmittel für Stoff zu verhindern.

16. Antitranspirationszusammensetzung nach Anspruch 17, wobei der Ölphasenbestandteil C12-15-Alkylbenzoat, Dimethicon, Polyethylen, Cetyl-PEG/PPG-10/Dimethicon, Bis-PEG/PPG-14/14-Dimethicon/Dimethicon, ein Polymer oder Kombinationen davon umfasst.

## Revendications

1. Composition antitranspirante avec de l'acide citrique comme agent réducteur de tâches, comprenant :
une émulsion eau dans huile comprenant :
un composant de phase huileuse disposé à l'intérieur de l'émulsion eau dans huile ; et
un composant de phase aqueuse disposé à l'intérieur de l'émulsion eau dans huile, le composant de phase aqueuse comprenant :
un ingrédient antitranspirant actif ; et
de l'acide citrique comme agent réducteur de tâches formant entre 0,9 à 4,0 pour cent en poids de l'émulsion eau dans huile,
l'émulsion eau dans huile ayant un pH entre 2,0 et 3,0.

2. Composition antitranspirante selon la revendication 1, dans laquelle l'agent réducteur de tâches à l'acide citrique protège l'ingrédient antitranspirant actif.

3. Composition antitranspirante selon la revendication 1, dans laquelle l'agent réducteur de tâches à l'acide citrique forme 1,5 pour cent en poids de l'émulsion eau dans huile.

4. Composition antitranspirante selon la revendication 1, dans laquelle l'agent réducteur de tâches à l'acide citrique empêche la polymérisation de l'ingrédient antitranspirant actif.

5. Composition antitranspirante selon la revendication 1, dans laquelle l'agent réducteur de tâches à l'acide citrique stabilise la distribution polymère de l'ingrédient antitranspirant actif, améliore la distribution polymère de l'ingrédient antitranspirant actif ou des combinaisons de ces derniers.

6. Composition antitranspirante selon la revendication 1, dans laquelle le composant de phase huileuse comprend un émollient, un certain nombre de supports, un certain nombre de structurants, un certain nombre d'émulsifiants ou des combinaisons de ces derniers.

7. Composition antitranspirante selon la revendication 1, dans laquelle l'ingrédient antitranspirant actif est un sel d'aluminium avec un pH entre 3,0 et 4,5.

8. Produit antitranspirant avec de l'acide citrique comme agent réducteur de tâches comprenant :
un récipient ; et
une émulsion antitranspirante eau dans huile logée à l'intérieur du récipient, l'émulsion antitranspirante eau dans huile comprenant :
un composant de phase huileuse disposé à l'intérieur de l'émulsion antitranspirante eau dans huile ; et
un composant de phase aqueuse disposé à l'intérieur de l'émulsion antitranspirante eau dans huile, le composant de phase aqueuse comprenant :
un ingrédient antitranspirant actif ; et
de l'acide citrique comme agent réducteur de tâches formant entre 0,9 à 4,0 pour cent en poids de l'émulsion eau dans huile, dans laquelle l'émulsion eau dans huile a un pH entre 2,0 et 3,0.

9. Produit antitranspirant selon la revendication 8, dans lequel l'agent réducteur de tâches à l'acide citrique protège l'ingrédient antitranspirant actif.

10. Produit antitranspirant selon la revendication 8, dans lequel le récipient comprend un distributeur pour pousser la composition antitranspirante solide hors du récipient.

11. Produit antitranspirant selon la revendication 8, dans lequel l'agent réducteur de tâches est configuré pour réduire la formation de précipité sur le tissu.

12. Produit antitranspirant selon la revendication 8, comprenant en outre un colorant, un parfum ou des combinaisons de ces derniers.

13. Produit antitranspirant selon la revendication 8, dans lequel l'agent réducteur de tâches à l'acide citrique forme 1,5 pour cent en poids de l'émulsion eau dans huile.

14. Composition antitranspirante selon la revendication 1, avec de l'acide citrique comme agent réducteur de tâches, comprenant :
une émulsion eau dans huile comprenant :
entre 25,0 et 35,0 pour cent en poids d'un composant de phase huileuse disposé à l'intérieur de l'émulsion eau dans huile ; et
entre 65,0 et 75,0 pour cent en poids d'un composant de phase aqueuse disposé à l'intérieur de l'émulsion eau dans huile, le composant de phase aqueuse comprenant :
un ingrédient antitranspirant actif ; et
de l'acide citrique comme agent réducteur de tâches pour protéger l'ingrédient antitranspirant actif formant entre 0,9 et 4,0 pour cent en poids de l'émulsion eau dans huile, l'émulsion eau dans huile ayant un pH entre 2,0 et 3,0.

15. Composition antitranspirante selon la revendication 17, dans laquelle l'agent réducteur à l'acide citrique est configuré pour empêcher une interaction entre l'ingrédient antitranspirant actif et un détergent pour tissu.

16. Composition antitranspirante selon la revendication 17, dans laquelle le composant de phase huileuse comprend du benzoate d'alkyle en c12 à 15, du diméthicone, du polyéthylène, du cétyl PEG/PPG-10/diméthicone, du bis-PEG/PPG-14/14 diméthicone/diméthicone, un polymère ou des combinaisons de ces derniers.
